(19) Europäisches Patentamt
European Patent Office
Office européen des brevets

(11) Veröffentlichungsnummer: **0 270 904**
**A2**

(12) # EUROPÄISCHE PATENTANMELDUNG

(21) Anmeldenummer: **87117281.3**

(22) Anmeldetag: **24.11.87**

(51) Int. Cl.4: **C07D 215/56**, C07D 471/04, C07D 498/06, //(C07D471/04,221:00,221:00),(-C07D498/06,265:00,221:00)

(30) Priorität: **05.12.86 DE 3641661**

(43) Veröffentlichungstag der Anmeldung:
**15.06.88 Patentblatt 88/24**

(84) Benannte Vertragsstaaten:
**BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Preiss, Michael, Dr.**
**Paul-Ehrlich-Strasse 12**
**D-5600 Wuppertal 1(DE)**

(54) **Verfahren zur Herstellung von Chinoloncarbonsäureestern.**

(57) Die vorliegende Erfindung betrifft ein Verfahren zur Herstellung von Chinoloncarbonsäureestern der Formeln I und II

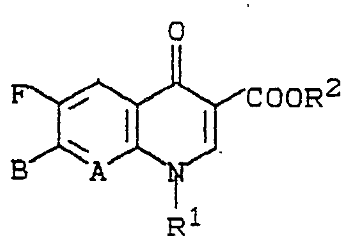

(I)

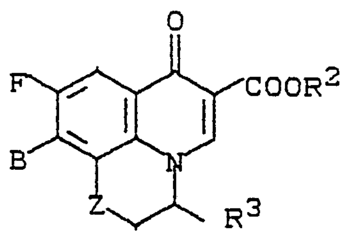

(II)

in denen

A für Stickstoff oder $=C-R^4$ steht,

$R^4$ für Wasserstoff, Fluor, Chlor, Nitro oder Methyl steht,

B für Halogen, Nitro, Alkoxy, Alkylsulfonyloxy oder den Rest

EP 0 270 904 A2

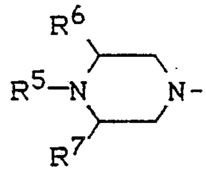

steht und B außerdem für

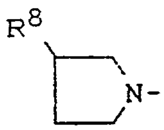

steht,

wenn $R^1$ nicht Cyclopropyl bedeutet und

$R^5$ für eine verzweigte oder unverzweigte Alkylgruppe mit 1 bis 4 Kohlenstoffatomen, die gegebenenfalls durch eine Hydroxy-oder Methoxygruppe substituiert sein kann, steht,

$R^6$ für Wasserstoff, Methyl oder Phenyl steht,

$R^7$ für Wasserstoff oder Methyl steht,

$R^8$ für Dialkylamino mit 1 oder 2 Kohlenstoffatomen in der Alkylgruppe oder Dialkylaminomethyl mit 1 oder 2 Kohlenstoffatomen in der Alkylgruppe oder Aminomethyl steht,

$R^1$ für Wasserstoff, substituiertes und unsubstituiertes Alkyl mit 1 bis 3 Kohlenstoffatomen, substituiertes und unsubstituiertes Cycloalkyl, 2-Fluorethyl, Vinyl, Methoxy oder 4-Fluorphenyl steht,

$R^2$ für gegebenenfalls substituiertes Alkyl mit 1 bis 6 Kohlenstoffatomen sowie Cyclohexyl, Benzyl steht,

$R^3$ für Wasserstoff, Methyl oder Ethyl steht,

Z für Sauerstoff, durch Methyl oder Phenyl substituierten Stickstoff sowie $=CH_2$ -steht,

das dadurch gekennzeichnet ist, daß man die entsprechenden Chinoloncarbonsäuren

mit Chlorameisensäureestern der Formel V

$Cl\text{-}COOR^2$ (V)

in welcher

$R^2$ die oben angegebene Bedeutung hat,

umsetzt.

BAYER AKTIENGESELLSCHAFT 5090 Leverkusen, Bayerwerk
Konzernverwaltung RP
Patentabteilung Rt/Ke-c

# Verfahren zur Herstellung von Chinoloncarbonsäureestern

Die vorliegende Erfindung betrifft ein Verfahren zur Herstellung von Chinoloncarbonsäureestern.

Die säurekatalysierte Veresterung von Carbonsäuren ist bekannt. Dabei wird die Carbonsäure in Gegenwart von Säuren mit Alkohol umgesetzt und das entstehende Wasser entweder azeotrop abdestilliert oder das Reaktionsgleichgewicht durch einen Überschuß an Alkohol verschoben. Im Fall der Chinoloncarbonsäuren sind dabei nur Ausbeuten von <50 % zu erreichen.

Basenkatalysierte Veresterungen von Chinoloncarbonsäuren z.B. durch Umsetzung der Säure in Gegenwart einer Base mit Alkylhalogenid ergeben zwar Ausbeuten von ca. 70 bis 80 %, erfordern jedoch den Einsatz kostspieliger reaktiver Alkylhalogenide, insbesondere Iodide, die zudem cancerogen sind.

Die Veresterung von Carbonsäuren mit Chlorameisensäureestern ist bekannt. Dabei entsteht jedoch als Nebenprodukt, vor allem bei der Umsetzung aromatischer Carbon-

säuren, das symmetrische Anhydrid (S. Kim et. al. Tetrahedron Letters 24 (1983) S. 3365-3368; A.L. Gutman et. al. Tetrahedron Letters 26 (1985) S. 1573-1576; (S. Kim et. al. J. Org. Chem. 1985(50) S. 560-565).

Es wurde nun gefunden, daß man Chinoloncarbonsäureester der Formeln I und II

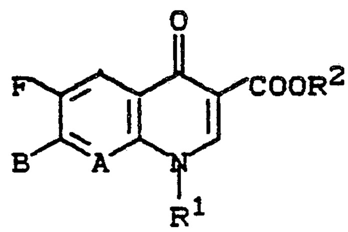

(I)

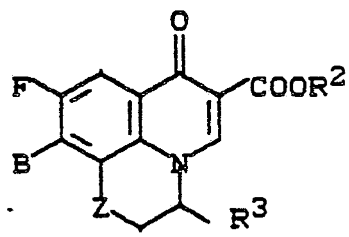

(II)

in denen

A für Stickstoff oder $=C-R^4$ steht,

$R^4$ für Wasserstoff, Fluor, Chlor, Nitro oder Methyl steht,

B für Halogen, Nitro, Alkoxy, Alkylsulfonyloxy oder den Rest

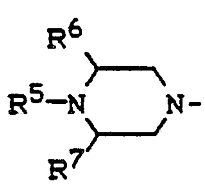 steht und B außerdem für 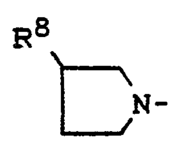 steht,

wenn $R^1$ nicht Cyclopropyl bedeutet und

$R^5$ für eine verzweigte oder unverzweigte Alkylgruppe mit 1 bis 4 Kohlenstoffatomen, die gegebenenfalls durch eine Hydroxy- oder Methoxygruppe substituiert sein kann, steht,

$R^6$ für Wasserstoff, Methyl oder Phenyl steht,

$R^7$ für Wasserstoff oder Methyl steht,

$R^8$ für Dialkylamino mit 1 oder 2 Kohlenstoffatomen in der Alkylgruppe oder Dialkylaminomethyl mit 1 oder 2 Kohlenstoffatomen in der Alkylgruppe oder Aminomethyl steht,

$R^1$ für Wasserstoff, gegebenenfalls substituiertes Alkyl mit 1 bis 3 Kohlenstoffatomen, gegebenenfalls substituiertes Cycloalkyl, 2-Fluorethyl, Vinyl, Methoxy oder 4-Fluorphenyl steht,

$R^2$ für gegebenenfalls substituiertes Alkyl mit 1 bis 6 Kohlenstoffatomen sowie Cyclohexyl, Benzyl steht,

$R^3$ für Wasserstoff, Methyl oder Ethyl steht,

Z für Sauerstoff, durch Methyl oder Phenyl substituierten Stickstoff sowie $=CH_2-$ steht,

erhält, indem man Chinoloncarbonsäuren der Formeln III und IV

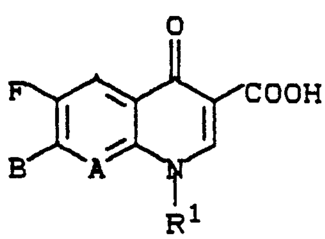

(III)

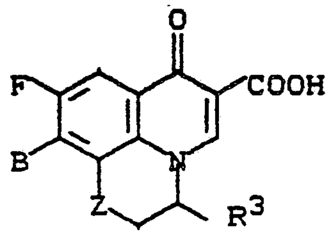

(IV)

in denen

A, B, Z, $R^1$, $R^3$ die oben angegebenen Bedeutungen haben,

mit Chlorameisensäureestern der Formel V

$$Cl\text{-}COOR^2 \qquad (V)$$

in welcher

$R^2$ die oben angegebene Bedeutung hat,

umsetzt.

Bevorzugt werden Chinoloncarbonsäureester der Formel (VI)

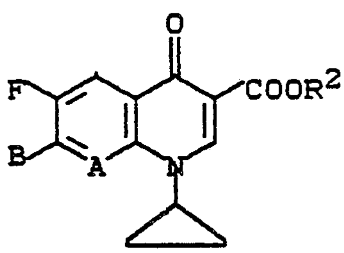

(VI)

in welcher

B für Fluor, Chlor oder den Rest

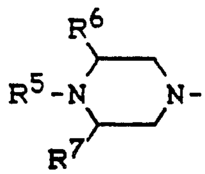 steht und

A, $R^2$, $R^5$, $R^6$, $R^7$ und $R^8$ die oben angegebene Bedeutung haben,

hergestellt.

Besonders bevorzugt werden Chinoloncarbonsäureester der Formel (VI)

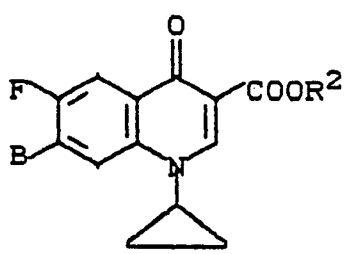

(VI)

in welcher

$R^2$ für Alkyl mit 1 bis 4 Kohlenstoffatomen, insbesondere Ethyl sowie Benzyl steht,

B für den Rest

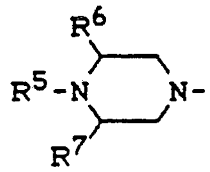 steht,

$R^5$ für Methyl oder Ethyl steht,

$R^6$ für Wasserstoff oder Methyl steht,

$R^7$ für Wasserstoff oder Methyl steht,

hergestellt.

Insbesondere werden die Methyl- oder Ethylester der folgenden Chinoloncarbonsäuren hergestellt:

1-Cyclopropyl-6-fluor-1,4-dihydro-4-oxo-7-(chlor oder fluor oder 4-methyl- oder 4-ethyl-1-piperazinyl-)chinolin-3-carbonsäure, 1-Ethyl-6-fluor-1,4-dihydro-4-oxo-7-(4-methyl-1-piperazinyl)-chinolin-3-carbonsäure, 1- Ethyl-6-fluor-1,4-dihydro-4-oxo-7-chlor-chinolin-3-carbonsäure, 1-Ethyl-6-fluor-1,4-dihydro-4-oxo-7-(4-ethyl-1-piperazinyl)-1,8-naphthyridin-3-carbonsäure, 9-Fluor-3-methyl-10-(4-methyl-1-piperazinyl)-7-oxo-2,3-dihydro-7,4-pyrido-[1,2,4-de]1,4-benzoxazin-6-carbonsäure.

Bevorzugt werden Chlorameisensäureester der Formel V eingesetzt in welcher $R^2$ für $C_{1-4}$-Alkyl steht, das gege-

benenfalls durch Phenyl, Halogen, OH substituiert ist. Besonders bevorzugt steht $R^2$ für $C_{1-4}$-Alkyl, insbesondere für Methyl oder Ethyl.

Als Basen seien genannt anorganische und organische Basen. Anorganische Basen sind Alkali- und Erdalkalihydroxide, -carbonate und -hydrogencarbonate. Organische Basen sind z.B. primäre, sekundäre und tertiäre Amine. Besonders bevorzugt sind tertiäre Amine. Als solche seien genannt:

Triethylamin, Pyridin, Picoline, Trimethylamin, N-Methylmorpholin, N-Ethylpyrrolidin, Diazabicyclo(4,3,0)-undecen (DBU), 1,4-Diaza-bicyclo-2,2,2-octan (DABCO), Diazabicyclo(3,2,0)nonen (DBN).

Die Reaktion wird in Gegenwart halogenierter aliphatischer Kohlenwasserstoffe mit 1 bis 4 C-Atomen und 1 bis 5 Halogenatomen durchgeführt. Als solche seien genannt z.B. Chloroform, Methylenchlorid, Dichlorethan, Trichlorethylen, Tetrachlorkohlenstoff. Besonders bevorzugt ist Chloroform.

Die Reaktion wird bei Temperaturen von -50° C bis 0° C durchgeführt, bevorzugt bei -20° C bis -15° C.

Die Reaktion wird bei Normaldruck durchgeführt.

Die Chlorameisensäureester der Formel V werden äquimolar oder in einem Überschuß von 5 bis 30 % bezogen auf die Chinoloncarbonsäuren der Formeln III und IV eingesetzt. Bevorzugt ist ein Überschuß von ca. 20 Gewichtsprozent.

Die Basen werden äquimolar bezogen auf die Chinoloncarbonsäuren der Formeln III und IV eingesetzt. Im Fall tertiärer organischer Amine ist es möglich, mit einem molaren Überschuß von 5 bis 30 %, bevorzugt 10 %, zu arbeiten.

Zur Durchführung der erfindungsgemäßen Reaktion werden die Chinoloncarbonsäuren der Formeln III und IV zunächst in wasserfreiem aliphatischem halogeniertem Kohlenwasserstoff gelöst und mit der Base versetzt. Die Mischung wird auf ca. -20° C gekühlt und bei dieser Temperatur unter Kühlen der Chlorameisensäureester zugetropft. Es wird bei -20° C nachgerührt und dann die Temperatur langsam auf Raumtemperatur erhöht. Danach wird die Reaktionsmischung mit Wasser versetzt und in üblicher Weise aufgearbeitet. Die Chinoloncarbonsäureester der Formeln I und II sind bekannte Bakterizide und Mikrobizide.

Beispiel 1

1-Cyclopropyl-6-fluor-1,4-dihydro-4-oxo-7-(4-ethyl-1-piperazinyl)-3-chinolincarbonsäureethylester

100 Gew.-Teile 1-Cyclopropyl-6-fluor-1,4-dihydro-4-oxo-7-(4-ethyl-1-piperazinyl)-3-chinolincarbonsäure wurden in 300 Vol.-Teilen trockenem Chloroform gelöst und mit 33,2 Gew.-Teilen Triethylamin versetzt. Dazu tropfte man bei -20° C innerhalb von 30 Minuten eine Lösung von 37,7 Gew.-Teilen Chlorameisensäureethylester in 100 Vol. Tlen. Chloroform, rührte noch 30 Minuten bei -20° C, dann über Nacht bei Raumtemperatur. Nach zweimaliger Extraktion mit je 300 Vol.-Teilen Wasser wurden zur organischen Phase 400 Vol.-Teile Wasser gegeben. Mit Eisessig wurde auf pH 5 gestellt (Verbauch ca. 15 Vol.-Teile) und das Chloroform im Wasserstrahlvakuum abdestilliert. Die wäßrige Lösung wurde mit halbkonz. Kalilauge auf pH 8,9 gestellt (Verbrauch ca. 70 Vol.-Teile) und über Nacht bei Raumtemperatur gerührt. Es wurde abgesaugt, mit 500 Vol.-Teilen Wasser gewaschen und bei 70° C im Vakuumtrockenschrank über Nacht getrocknet.

| | |
|---|---|
| Ausbeute: | 103,2 Gew.-Teile (95,7 % der Theorie), Fp. 183 bis 185° C |
| Gehalt (HPLC): | 98,8 Fl.-% |

Patentansprüche

1. Verfahren zur Herstellung von Chinoloncarbonsäureestern der Formeln I und II

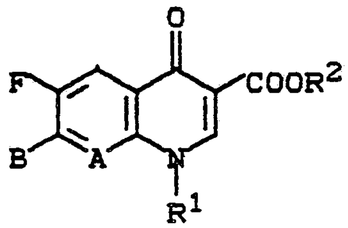

(I)

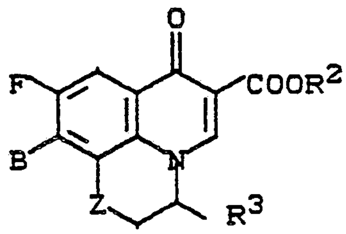

(II)

in denen

A für Stickstoff oder =C-$R^4$ steht,

$R^4$ für Wasserstoff, Fluor, Chlor, Nitro oder Methyl steht,

B für Halogen, Nitro, Alkoxy, Alkylsulfonyloxy oder den Rest

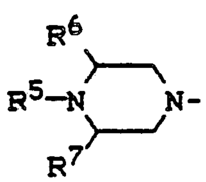 steht und B außerdem für 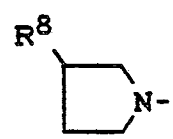 steht,

wenn $R^1$ nicht Cyclopropyl bedeutet und

$R^5$ für eine verzweigte oder unverzweigte Alkylgruppe mit 1 bis 4 Kohlenstoffatomen, die gegebenenfalls durch eine Hydroxy- oder Methoxygruppe substituiert sein kann, steht,

$R^6$ für Wasserstoff, Methyl oder Phenyl steht,

$R^7$ für Wasserstoff oder Methyl steht,

$R^8$ für Dialkylamino mit 1 oder 2 Kohlenstoffatomen in der Alkylgruppe oder Dialkylaminomethyl mit 1 oder 2 Kohlenstoffatomen in der Alkylgruppe oder Aminomethyl steht,

$R^1$ für Wasserstoff, gegebenenfalls substituiertes Alkyl mit 1 bis 3 Kohlenstoffatomen, gegebenenfalls substituiertes Cycloalkyl, Vinyl, Methoxy oder 4-Fluorphenyl steht,

$R^2$ für gegebenenfalls substituiertes Alkyl mit 1 bis 6 Kohlenstoffatomen sowie Cyclohexyl, Benzyl steht,

$R^3$ für Wasserstoff, Methyl oder Ethyl steht,

Z für Sauerstoff, durch Methyl oder Phenyl substituierten Stickstoff sowie $=CH_2-$ steht,

dadurch gekennzeichnet, daß man Chinoloncarbonsäuren der Formeln III und IV

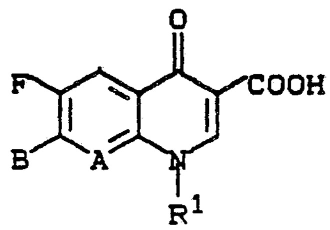

(III)

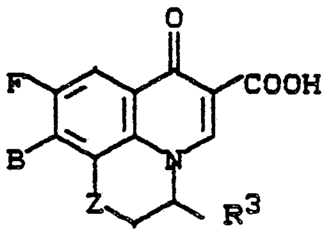

(IV)

in denen

A, B, Z, $R^1$, $R^3$ die oben angegebenen Bedeutungen haben,

mit Chlorameisensäureestern der Formel V

$$Cl\text{-}COOR^2 \qquad (V)$$

in welcher

$R^2$ die oben angegebene Bedeutung hat,

umsetzt.